# EUROPEAN PATENT APPLICATION

(11) **EP 1 459 734 A1**
(43) Date of publication of application: **22.09.2004**
(21) Application number: 04075852.6
(22) Date of filing: 17.03.2004
(51) Int. Cl.: A61K 7/40

(54) **Cosmetic product containing an active phytotherapy agent and method of manufacture thereof**

(30) Priority: 20.03.2003 BR 0301167
(71) Applicant: V.F. Franqueadora de Farmacia e Manipulacao, Ltd., CEP 03119-000 Sao Paulo-SP (BR)
(72) Inventor: Amaral, Everson, Santo André - SP CEP-09181-58 (BR); dos Santos Filho, Antonio José, Ribeirao Pires - SP CEP - 09421-000 (BR)
(74) Representative: De Magalhaes Simoes, José Raul

(57) **Abstract**

Cosmetic products containing an active phytotherapy agent and manufacture thereof. Cosmetic products containing an active agent of vegetable origin and manufacture thereof, in particular Lutein which, after being industrially processed with other constituent elements in varying concentrations with topical application, with the therapeutic actions intended, which, moisturise the skin corporal area, hands and face.

## Description

### TECHNICAL FIELD

The present invention regards cosmetic products containing an active principle from vegetable origin and its respective obtaining process, in particularly the Lutein, which after industrially processed with other constituent elements allows the achievement of varied concentrations with topical application, with the therapeutic actions intended, which are nutritious lotion for the skin corporal area, hands and facial.

### DESCRIPTION OF THE TECHNIQUE

The great majority of the people, mainly doctors dedicated to study the subject of the invention in matter, known that the human skin works its shield against the harmful effect of the environment, and the solar radiation, along with others the pollution is the main detonator agent of countless problems, that go from precocious aging to skin cancer.

The ultraviolet rays UV-A and UV-B have an extremely malicious effect on the skin It is considered that 80% of the wrinkles, stains and fine wrinkles result from the lingering exhibition to solar radiation, and that the 20 UV-B rays are responsible for the largest incidence of skin cancer, attacking the immunological system and reducing the defence capacity of the organism, while the UV-A rays, that at first could seem more harmless, are the main cause of the precocious aging, and with cumulative effect, it can provoke stains and wrinkles with the passing of the years.

As it is of general knowledge, Lutein is a member of the carotenoids family and it is found in fruits and vegetables of green leaves, such as spinaches and broccolis. Lutein contains an antioxidant element which can protect the skin against the stress oxidizer of the UV-A and UV-B rays, being, therefore, quite effective in the help against the processes related with the aging of the skin.

### DESCRIPTION OF THE INVENTION

It is the objective of this invention to present phytotherapeutic substances using the Lutein as an active principle, which associated mainly with vitamins E and C and with topical application, present proved antioxidant action in the human body.

A first composition refers to a corporal nutritious Lotion, comprehending the following components:

| Phase - A | |
|---|---|
| Unionised Water | 80,32%. |
| Propyleneglycol | 3,00%; |
| Glycerin | 3,00%; |
| Carbopol Gel | 2,00%; |

| Phase - B | |
|---|---|
| Self emulsion Wax | 6,00%; |
| Triglycerides of Capric Acid | 4,00%, |
| Lutein and Carotene | 0,08%; |

| Phase - C | |
|---|---|
| Phenoxyethanol and Polymer of beta-hydroxybutyrate (PHB) | 0,30%; |
| Urea Imidazolidinyl | 0,20%; |
| Essence | 0,60%; |
| Vitamin and oily | 0,50%; |

A second composition refers to a facial nutritious Cream with Lutein - Carotenoid and Vitamin E:

| Phase - A | |
|---|---|
| Unionised Water | 81,30%. |
| Triglycerides of Capric Acid | 4,00%; |

| Phase - B | |
|---|---|
| Self emulsion Wax | 10,00%; |
| Propyleneglycol | 3,00%; |
| Lutein and Carotene | 0,10%, |

| Phase - C | |
|---|---|
| Phenoxyethanol and Polymer of beta-hydroxybutyrate (PHB) | 0,30%; |
| Urea Imidazolidinyl | 0,20%; |
| Essence | 0,60%; |
| Vitamin and oily | 0,50%; |

A third composition refers to a Composition: Cream for hands, Lutein - Carotenoid and Vitamin E

| Phase - A | |
|---|---|
| Unionised Water | 83,92%. |
| Propyleneglycol | 3,00%; |

| Phase - B | |
|---|---|
| Self emulsion Wax | 7,00%; |
| Mineral oil | 0,50%; |
| Triglycerides of Capric Acid | 4,00%; |
| Lutein and Carotene | 0,08%; |

| Phase - C | |
|---|---|
| Phenoxyethanol and Polymer of beta-hydroxybutyrate (PHB) | 0,30%; |
| Urea Imidazolidinyl. | 0,20%; |
| Essence | 0,50%; |
| Vitamin and oily | 0,50%; |

The process of obtaining all of the above described products follows the same following steps.
1) Add in tank 2 the phase (A), heat up to 70 - 75°C; agitation turbine helix 3 minutes;
2) Add in tank 3 the phase (B), heat up to 70 - 75°C for 10 minutes;
3) Add B in the phase (A) in the tank 3 with turbine / helix agitation;
4) Let emulsify, with slow agitation of turbine, the mass of the tank for 3 minutes or until it completes dissolution;
5) Verify the temperature which should have been among 70 - 75°C, and to adjust if necessary.
6) Let it agitate for 10 minutes at 30 RPM;
7) Remove at least 2 recipients from the bottom, and put them on top to equal the temperatures;
8) Cool the temperature to 45°C;
9) Add the phase (C) in the sequence and agitate it for 5 minutes.

In spite of the detail of the invention, it is important to understand that the same doesn't limit its application to the details and stages herein described. The invention is capable of other forms and of being practiced or executed in a variety of manners. It should be understood that the terminology here employed it is for the description purpose and is not of limitation.

## Claims

1. " COSMETIC PRODUCTS CONTAINING ACTIVE PHYTOTHERAPY PRINCIPLE AND RESPECTIVE OBTAINING PROCESS", **characterized by** the phytotherapy products or substances with topical application using Lutein as an active principle associated mainly with the vitamins E and C, presenting antioxidant action of the human skin.

2. "PRODUCT", in agreement with claim 1 and in a first composition, **characterized by** the corporal nutritious lotion to comprehend the following components:
| Phase - A | |
|---|---|
| Unionised Water | 80,32% |
| Propyleneglycol | 3,00%, |
| Glycerin | 3,00%; |
| Gel Carbopol | 2,00%; |
| Phase - B | |
|---|---|
| Self emulsion Wax | 6,00%; |
| Triglycerides of Capric Acid | 4,00%; |
| Lutein and Carotene | 0,08%; |
| Phase - C | |
|---|---|
| Phenoxyethanol and Polymer of beta-hydroxybutyrate (PHB) | 0,30%; |
| Urea Imidazolidinyl | 0,20%; |
| Essence | 0,60%, |
| Vitamin and oily | 0,50%, |

3. "PRODUCT", in agreement with claim 1 and in a second composition, **characterized by** the facial nutritious cream comprising the following components:
| Phase - A | |
|---|---|
| Unionised Water | 81,30% |
| Triglycerides of Capric Acid | 4,00%, |
| Phase - B | |
|---|---|
| Self emulsion Wax | 10,00%, |
| Propyleneglycol | 3,00%, |
| Lutein and Carotene | 0,10%; |
| Phase - C | |
|---|---|
| Phenoxyethanol and polymer of beta-hydroxybutyrate (PHB) | 0,30%; |
| Urea Imidazolidinyl | 0,20%, |
| Essence | 0,60%; |
| Vitamin and oily | 0,50%; |

4. "PRODUCT", in agreement with claim 1 and in a third composition, **characterized by** the cream for hands comprising the following components:
| Phase - A | |
|---|---|
| Unionised Water | 83,92%. |
| Propyleneglycol | 3,00%; |
| Phase - B | |
|---|---|
| Self emulsion Wax | 7,00%; |
| Mineral oil | 0,50%, |
| Triglycerides of Capric Acid | 4,00%; |
| Lutéina and Carotene | 0,08%; |
| Phase - C | |
|---|---|
| Phenoxyethanol and polymer of beta-hydroxybutyrate (PHB) | 0,30%, |
| Urea Imidazolidinyl | 0.20%; |
| Essence | 0,50%; |
| Vitamin and oily | 0,50%; |

5. " OBTAINING PROCESS ", in agreement with the previous claims, **characterized by** the obtaining process to carry out the following steps
1) Add in tank 2 the phase (A), heat up to 70 - 75°C agitation turbine helix 3 minutes;
2) Add in tank 3 the phase (B), heat up to 70 - 75°C for 10 minutes;
3) Add B in the phase (A) in the tank 3 with turbine / helix agitation;
4) Let emulsify, with slow agitation of turbine, the mass of the tank for 3 minutes or until it completes dissolution;
5) Verify the temperature which should have been among 70 - 75°C, and to adjust if necessary,
6) Let it agitate for 10 minutes at 30 RPM;
7) Remove at least 2 recipients from the bottom and put them on top to equal the temperatures;
8) Cool the temperature to 45°C;
9) Add the phase (C) in the sequence and it agitate for 5 minutes.
